# EUROPEAN PATENT SPECIFICATION

(11) **EP 4 598 993 B1**
(45) Date of publication and mention of the grant of the patent: **02.12.2026**
(21) Application number: 23785809.7
(22) Date of filing: 06.10.2023
(51) Int. Cl.: C08J 11/16, C08J 11/28

(54) **BASE-MEDIATED DECONSTRUCTION OF EPOXY RESINS**
BASENVERMITTELTE DEKONSTRUKTION VON EPOXIDHARZEN
DÉCONSTRUCTION DE RÉSINES ÉPOXYDES MÉDIÉE PAR UNE BASE

(30) Priority: 06.10.2022 EP 22200007
(43) Date of publication of application: 13.08.2025
(73) Proprietor: Aarhus Universitet, 8000 Aarhus C (DK)
(72) Inventor: SUN, Hongwei, 8000 Aarhus C (DK); AHRENS, Alexander, 8000 Aarhus C (DK); SKRYDSTRUP, Troels, 8000 Aarhus C (DK)
(74) Representative: Høiberg P/S
(86) International application number: PCT/EP2023/077770
(87) International publication number: WO 2024/074703

(56) References cited:
- US-A1- 2019 203 013

## Description

### Technical field

The presented invention relates to a method for disassembly of epoxy-based polymers, the method comprising chemical manipulations to a cured epoxy resins involving a mixture of at least one apolar aprotic organic solvent and a base as defined in the claims. Specifically, the epoxy-based polymer is an amine-cured epoxy resin comprising bisphenol constituents. The present invention allows for recycling of the constituents comprised in the cured epoxy resin in high yields, notably even in the absence of any catalyst, metal or otherwise.

### Background

The sheer amount of end-of-use plastics and plastic-containing materials released into Nature has resulted in a major environmental crisis, strongly affecting ecosystems across the globe. The need for the implementation of a circular economy of plastics and plastic-containing composites, reducing the consumption of resources, as well as limiting the introduction of waste into the environment, has become apparent.

Chemical recycling has the ability to disassemble otherwise end-of-use polymers into their original monomers or related base chemicals, which can then re-enter production chains yielding virgin-grade polymeric materials. Thereby, enabling such a circular economy holds the opportunity of turning the accumulating plastic waste into valuable resources. Recently, the catalytic hydrogenation of carbonyl moieties in thermoset polyurethanes, recovering both anilines and polyols, has been reported as a strategy realizing this principle. In contrast, epoxy resins lack reactive carbonyl moieties, making selective disconnections of their chemical bonds more challenging. Therefore, without a viable recycling technology at hand, the value and potential of epoxy resins in fibre-reinforced epoxy composites comes together with an aftertaste of unsustainability.

US 2019/203013 A1 discloses methods for degradation of fibre-reinforced epoxy composites using transition metal catalysts.

Fibre-reinforced epoxy composites are light weight, highly durable materials which usually consist of glass or carbon fibres embedded in epoxy-based polymer matrices, and are crucial to the construction of automobiles, boats, airliners and wind turbine blades. In 2021, the wind turbine producer Siemens Gamesa commercialised turbine blades based on a resin system that allows the separation of fibers from resins using acidic conditions. While elegant, the reuse of the recovered epoxy fraction is limited only to uses such as a filler material, meaning that circular solutions have not been achieved yet.

With society moving towards reducing or banning landfilling, there is clearly a need in the art for improved methods to deconstruct epoxy resins via chemical processes, especially those methods that allow recovery and recycling of the base constituents.

### Summary

The invention is defined by the claims. Any subject matter falling outside the scope of the claims is provided for information purposes only.

The present disclosure is directed to a method for base-mediated disassembly of epoxy-based polymers (EPBs) via chemical manipulations involving organic solvents or mixtures thereof and a suitable base as described herein, thereby allowing for recycling of the constituents of EBPs which would otherwise largely be deposited in landfills or incinerated for energy recovery, creating air pollution. The use of metal catalysts for industrial scale applications, especially those catalysts based on expensive transition metals such as ruthenium, palladium, rhodium, platinum and gold, is highly undesirable as it often renders an otherwise useful reaction unfeasible from the financial point of view. Furthermore many of these catalytic processes require exclusion of moisture and/or oxygen, which makes upscaling more challenging and impedes fast process developments for commercial applications. Thus, the method for base-mediated disassembly of epoxy-based polymers described herein does not involve the use of a transition metal catalyst.

A major aspect of the present invention is a method for the deconvolution of epoxy-based polymers (EBPs), the method comprising the steps:
a. providing an EBP,
b. contacting the EBP with a solvent system comprising at least one aprotic apolar organic solvent and an oxygen-containing base to form a suspension, and
c. heating said suspension to a temperature of at least 130°C for at least 2 hours, thereby releasing monomers of the EBPs into the suspension,
wherein the base is added in an amount corresponding to from 20 % to 100 % by weight of the EBP, and wherein the method does not comprise or make use of a transition metal catalyst.

It is preferred that said steps a, b, and c of the method are performed sequentially in the specified order.

The EBPs as described within the present disclosure may comprise the chemical motif of Formula (III-a): 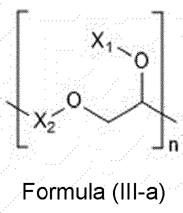 wherein,
X₁ is selected from the group consisting of H, R, or -C(=O)R, wherein R can be C₁₋₆ alkyl, C₁₋₆ heteroalkyl, C₁₋₆ alkoxy, C₁₋₆ allyl, C₁₋₆ heteroallyl, C₄₋₈ cycloalkyl, C₄₋₈ heterocycloalkyl, C₄₋₈ aryl, and C₄₋₈ heteroaryl, each of which may be optionally substituted with one or more of -OH, -NH₂, -L, -CL₃, -OCH₃, wherein L is a halogen;
X₂ is selected from the group consisting of C₄₋₈ cycloalkyl, C₄₋₈ heterocycloalkyl, C₄₋₈ aryl, and C₄₋₈ heteroaryl, each of which may be optionally substituted with one or more of - OH, -NH₂, -Q, -CQ₃, -OCH₃, wherein each of Q is independently hydrogen, halogen, methyl, phenyl or benzyl; and
n represents the motif as repeating, and may be an integer larger than 1.

### Description of Drawings

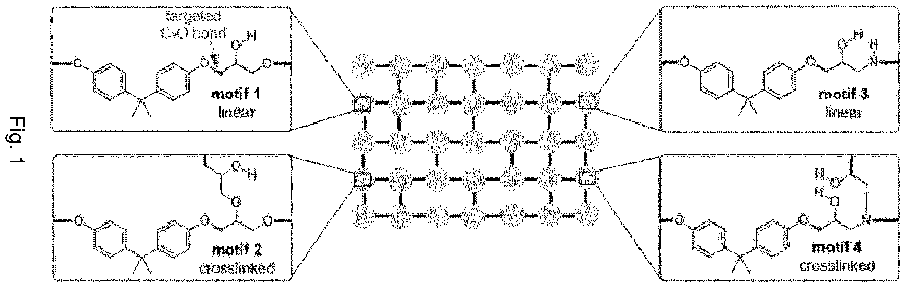

**Figure 1****:** Idealized schematic figure of extended network in epoxy-based polymers based on a monomer of Bisphenol A. The schematic further highlights the specific bonds which are envisioned as broken in deconvolution of the epoxy-based polymer into its base constituents.

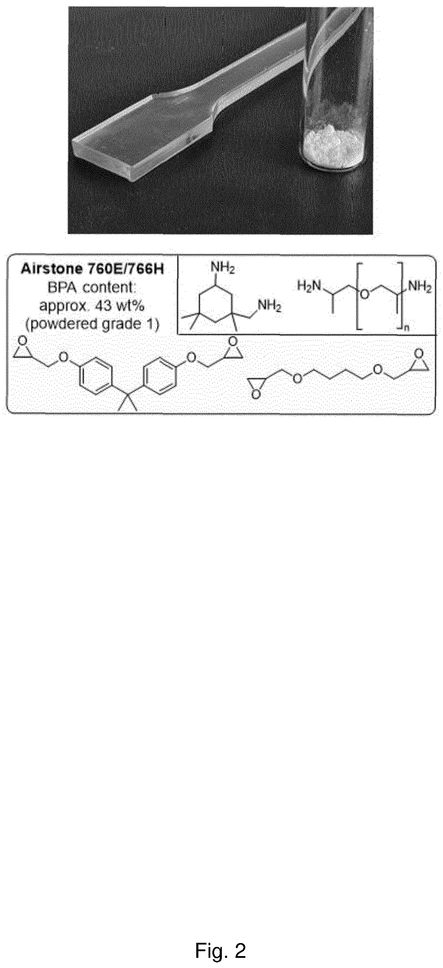

**Figure 2****:** Picture of commercially available epoxy-based polymer Airstone^{™} 760E/766H and its base constituents as deconvoluted in Examples 3 to 6. Shown at the bottom are the epoxy-monomers functionalized with diglycidyl ether, and at the top the curing agents, also referred to as epoxy-hardeners.

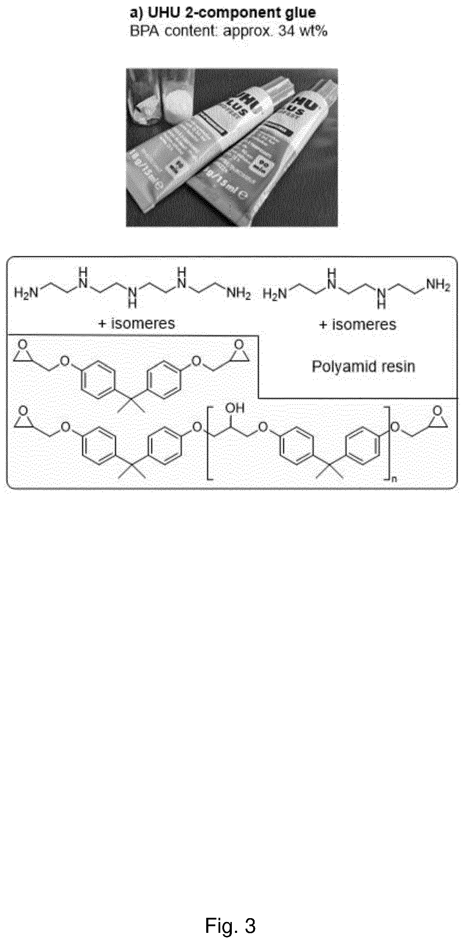

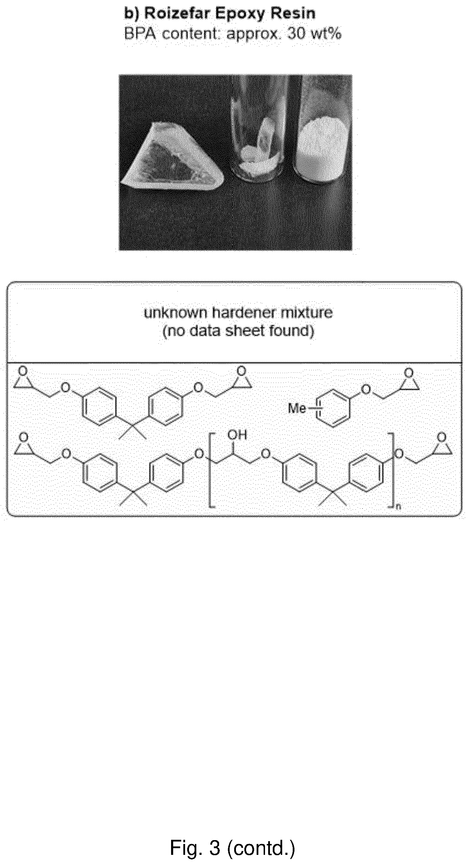

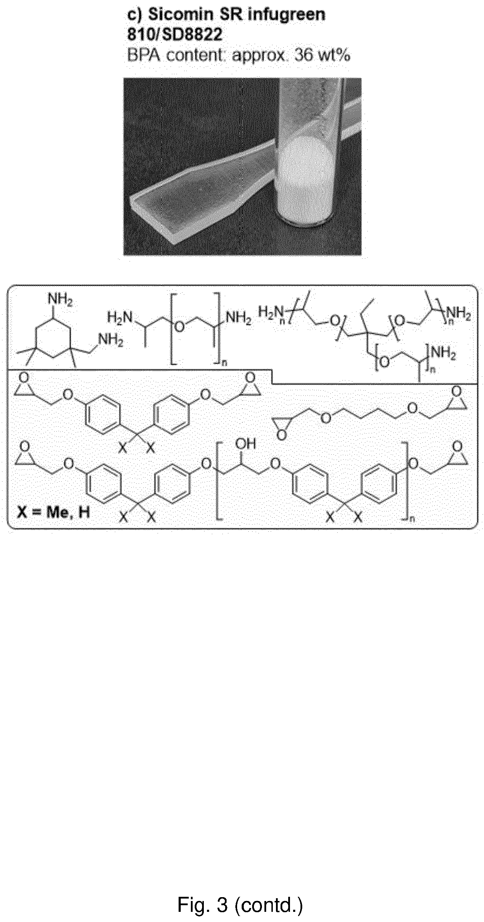

**Figure 3****:** Pictures of commercially available epoxy-based polymers and its base constituents as deconvoluted in Example 4. Shown at the bottom are the epoxy-monomers functionalized with diglycidyl ether, and at the top the curing agents, also referred to as epoxy-hardeners. a) UHU 2-component glue; b) Roizefar Epoxy Resin; and c) Sicomin SR infugreen 810/SD8822.

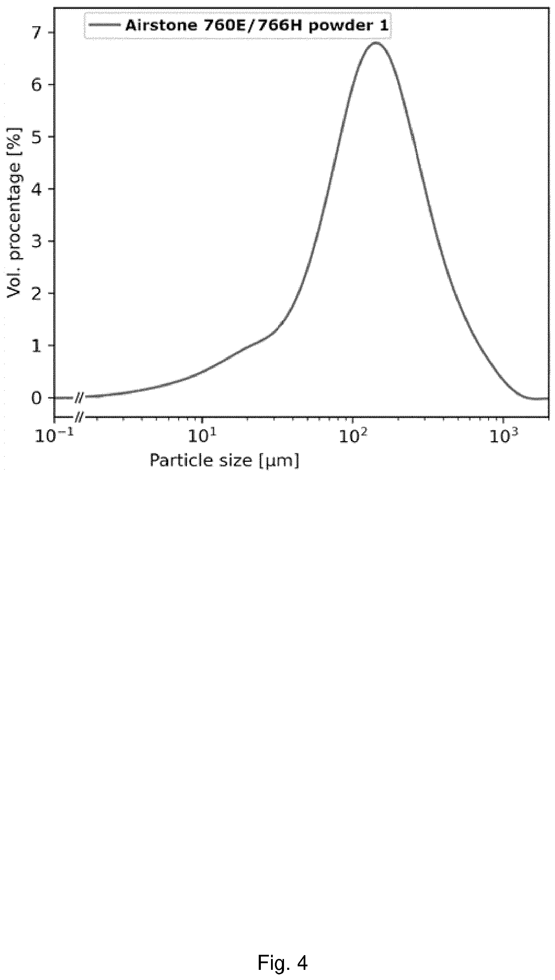

**Figure 4****:** Distribution of particle sizes in filed Airstone^{™} 760E/766H powder as determined by dynamic light scattering (described in Examples 1 and 3). Dynamic light scattering measurements revealed that 10% of the particles have a diameter below 32.8 µm, 50% are above and 50% below 134.1 µm in diameter and 90% have a diameter below 380.5 µm.

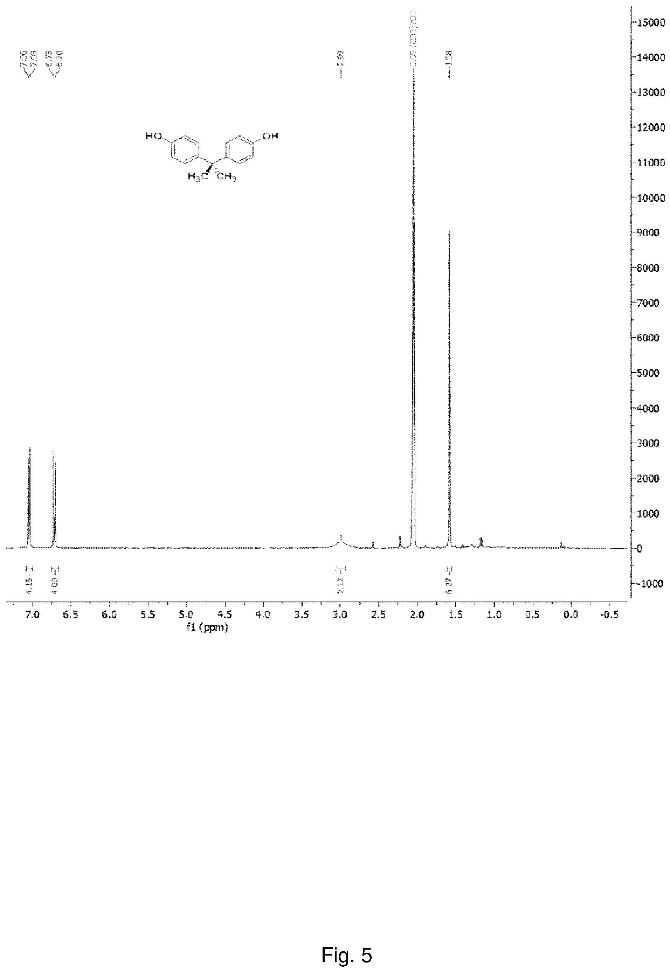

**Figure 5****:** ¹H-NMR of crude Bisphenol A as obtained by process ii) of Example 5. The spectrum is measured in deuterated acetone (CD₃)₂CO, and the peak of deuterated acetone identified at 2.05 ppm. The image shows all identified peaks but is otherwise cropped for size considerations. Chemical shift range indexed from -0.5 to 7.0 in major increments of 0.5. signal count indexed from -1.000 to 15.000 in major increments of 1.000. Further peak identifiers are given in Example 5.

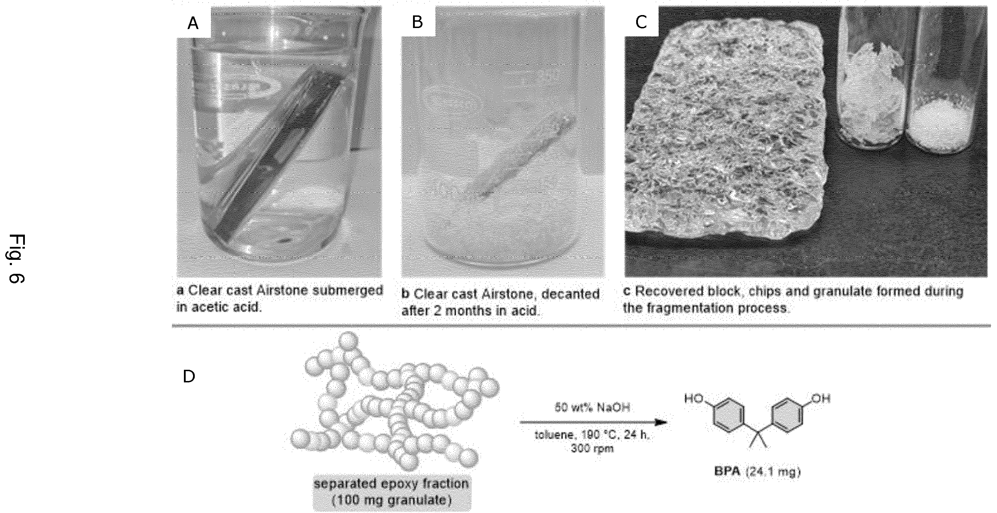

**Figure 6****:** pictures and reaction schematic of the deconstruction process utilized in Example 6. 6A: clear cast Airstone^{™} 760E/766H submerged in acetic acid. 6B: decanted Airstone resin after two months in acetic acid showing that the resin has been broken apart. 6C: recovered fragments after decanting (from left: block, chips and granulate). The granulate sample was used 'as-is' for the purpose of Example 6. 6D: reaction schematic of the deconstruction taking place in Example 6.

### Definitions

The term "polymer" as used herein refers to any of a class of substances composed of multiple linked repeating units, such as repeating molecules.

The term "EBPs" as used herein refers to epoxy-based polymers. In the present context, these are polymeric and/or cross-linked materials formed by the reaction of substances containing epoxide functional groups with themselves or with other co-reactants. The epoxide functional group is collectively called epoxy.

The term "FREBPs" as used herein refers to fibres reinforced with epoxy-based polymers. In the present context, these are composite materials comprising fibres or other materials of different origin embedded in an epoxy-based polymer matrix.

The term "fibre" as used herein refers to any non-spherical material which may advantageously be cured together with an epoxy-based polymer to improve the structural and/or durable integrity of the composite material, compared to the cured polymer without the presence of the fibre material. Non-limiting examples of fibre materials within the meaning of the present disclosure are glass, cellulose, plastic, steel, metal, carbon, and more. Fibre and fiber may have been used interchangeably herein.

The term "monomer" as used herein refers to molecules or any class of compounds that can react with itself or other molecules to form extended structures, such as polymers. Also as used herein, "monomer" may refer to molecules forming part of the repeating unit of a polymer. Preferably monomers are largely organic molecules comprising suitable functionalities known to a person skilled in the art. When referring to monomer within the context of the present invention, it is intended to refer to a chemical entity which may have been functionalized with epoxides for use in forming epoxy-based polymers. This includes without limitation bisphenols derivatized with electrophilic epoxides under which circumstances, the monomer would refer to the non-derivatized bisphenol.

The term "cross-linker" as used herein refers to molecules or any class of compounds that can react to connect two or more polymer chains, as well as the chemical structures connecting two or more polymer chains in a network. Also as referred to herein, "crosslinker" refers to molecules which may react with epoxide-functionalized entities such as bisphenol derivatives to form epoxy-based polymers.

The term "deconvolution" as used herein refers generally to manipulation of a material ABCD into at least some of its base constituents A, B, C, and D, thereby allowing their individual recovery. As used herein, "deconvolution" is used interchangeably with "deconstruction", "disassembly", "disconnection" and "degradation".

The term "catalyst" as used herein refers to any substance that increases the rate of a chemical reaction or reactions without itself undergoing any permanent chemical change. In addition, the term catalyst is a term well known to those skilled in the field.

### Detailed description

The present disclosure is directed to a new method for base-mediated disassembly of epoxy-based polymers (EPBs) via chemical manipulations involving organic solvents or mixtures thereof and a suitable base as described herein, thereby allowing for recycling of the constituents of EBPs which would otherwise largely be deposited in landfills or incinerated to recover energy while creating undesired air pollution.

One embodiment of the present disclosure is to provide the method herein described for the deconvolution of EBPs via degradation of chemical linkages in the polymer matrix.

The present inventors have surprisingly identified a composition mixture and temperature window where EPBs can be deconstructed very efficiently, using only simple reagents to achieve high yields of base constituents such as bisphenols, more preferably Bisphenol A. It is even more surprising that the extremely high efficiency as demonstrated herein can be achieved even without the need of a catalyst, such as without the need of a metal catalyst or transition metal catalyst. Furthermore the developed protocol has been demonstrated to be tolerant towards oxygen (air) and moisture. Without wishing to be bound by theory, the present inventors contemplate the mismatch between a base and an aprotic apolar organic solvent to be key for inducing base-mediated C-O bond cleavage because the poorly solvated ions would be much more reactive in targeting relevant bonds than if they were solubilized. Likewise, ionic or polar intermediates formed during epoxy transformation into monomers would be equally affected by the apolar medium thereby enabling challenging bond cleavages to take place under relatively mild conditions.

From the reaction mixture it is therefore possible to directly isolate monomers of bisphenols, such as the base constituents used to make the EBPs including before any derivatization with diglycidyl ether. Therefore, one embodiment of the presently disclosed method is wherein the method is a one-pot synthesis. The limitations and advantages of one-pot synthesis is well-known in the chemical community and to persons of skill in the art. Thus in one embodiment of the present disclosure, the monomers released into the suspension when working the disclosed method are bisphenol-based monomers such as bisphenol A-based monomers, more preferably non-derivatized bisphenol A monomers.

One embodiment of the present disclosure is the method for the deconvolution of epoxy-based polymers (EBPs) disclosed herein, the method comprising providing an EBP and further comprising a step of contacting the EBP with a solvent system comprising at least one organic solvent and a base to form a suspension.

The method herein disclosed, is performed in the absence of a transition metal catalystsuch as no transition metal catalyst is added at any point of the deconstruction of the epoxy-based polymer.

It is within the scope of the present disclosure to provide the methods disclosed herein for deconvolution of EBPs including those found in FREBPs allowing recovery of the EBP base constituents, most preferably the provided methods are for deconvolution of polymers obtained from using a Bisphenol diglydicyl ether as a starting material, exemplary Bisphenol A diglycidyl ether:

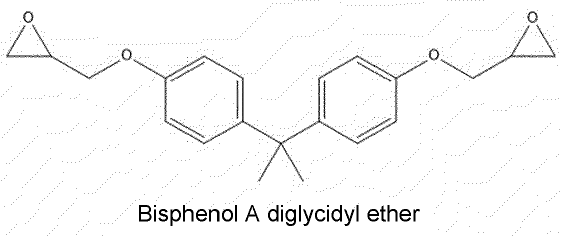

In such embodiments, one of these recovered constituents is bisphenol A (BPA). BPA originates from unsustainable petrochemicals and is a major building block for the production of polymers with 6 million metric tons being produced in 2017 alone. For the preparation of epoxy resins, BPA is functionalized with electrophilic epoxide moieties such as diglycidyl ethers. These mono- or oligomeric epoxides can then be cured with exemplary (multifunctional) alkyl amines, yielding randomized 3D polymer networks, knitted together by strong C-O and C-N sigma bonds in different linkages, such as schematically illustrated in Fig. 1. It is also within the scope to expand this methodology to other epoxy-derivatized bisphenol compounds, such as those bisphenol derivatives known to persons of ordinary skill in the art.

In one embodiment of the method disclosed herein the EBPs comprise the chemical motif of formula (II-a) or formula (II-a1): 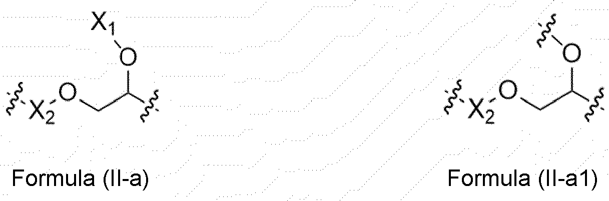 wherein,
X₁ can be H, R, or -C(=O)R, wherein R can be C₁₋₆ alkyl, C₁₋₆ heteroalkyl, C₁₋₆ alkoxy, C₁₋₆ allyl, C₁₋₆ heteroallyl, C₄₋₈ cycloalkyl, C₄₋₈ heterocycloalkyl, C₄₋₈ aryl, and C₄₋₈ heteroaryl, each of which may be optionally substituted with one or more of -OH, -NH₂, -L, -CL₃, -OCH₃, wherein L is a halogen, and
X₂ can be C₄₋₈ cycloalkyl, C₄₋₈ heterocycloalkyl, C₄₋₈ aryl, and C₄₋₈ heteroaryl, each of which may be optionally substituted with one or more of -OH, -NH₂, -Q, -CQ₃, -OCH₃, wherein each of Q is independently hydrogen, halogen, methyl, phenyl or benzyl. Preferably, in formula II-a or formula II-a1, X₂ is a C₄₋₈ aryl, preferably an aromatic C₆ aryl such as benzene, optionally substituted with one or more of -OH, -NH₂, -Q, -CQ₃, - OCH₃, wherein each of Q is independently hydrogen, halogen, methyl, phenyl or benzyl.

Thus, in one embodiment of the method disclosed herein the EBPs comprise the chemical motif of formula (III-a): 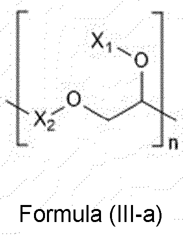 wherein,
X₁ can be H, R, or -C(=O)R, wherein R can be C₁₋₆ alkyl, C₁₋₆ heteroalkyl, C₁₋₆ alkoxy, C₁₋₆ allyl, C₁₋₆ heteroallyl, C₄₋₈ cycloalkyl, C₄₋₈ heterocycloalkyl, C₄₋₈ aryl, and C₄₋₈ heteroaryl, each of which may be optionally substituted with one or more of -OH, -NH₂, -L, -CL₃, -OCH₃, wherein L is a halogen, and
X₂ can be C₄₋₈ cycloalkyl, C₄₋₈ heterocycloalkyl, C₄₋₈ aryl, and C₄₋₈ heteroaryl, each of which may be optionally substituted with one or more of -OH, -NH₂, -Q, -CQ₃, -OCH₃, wherein each of Q is independently hydrogen, halogen, methyl, phenyl or benzyl, and n represents the motif as repeating, and may be an integer larger than 1.

Preferably, X₁ in formula (III-a) is H, and X₂ is a C₄₋₈ aryl, preferably an aromatic C₆ aryl such as benzene, optionally substituted with one or more of -OH, -NH₂, -Q, -CQ₃, -OCH₃, wherein each of Q is independently hydrogen, halogen, methyl, phenyl or benzyl.

In one embodiment of the present disclosure, n as defined in Formula (III-a) is an integer of at least 1, such as at least 2, such as at least 5, such as at least 10, such as at least 50, such as at least 100, such as at least 200, such as at least 500, such as at least 1.000, such as at least 10.000.

In one embodiment of the present disclosure, n as defined in Formula (III-a) is an integer between 1 and 1000, such as between 1 and 500, such as between 1 and 400, such as between 1 and 300, such as between 1 and 200, such as between 1 and 100.

In one embodiment of the present disclosure, n as defined in Formula (III-a) is an integer between 50 and 100, such as between 100 and 200, such as between 200 and 300, such as between 300 and 500, such as between 500 and 700, such as between 700 and 1000.

In one embodiment of the method disclosed herein the EBPs comprise the chemical motif of formula II-a(i) or formula II-a(i)-1: 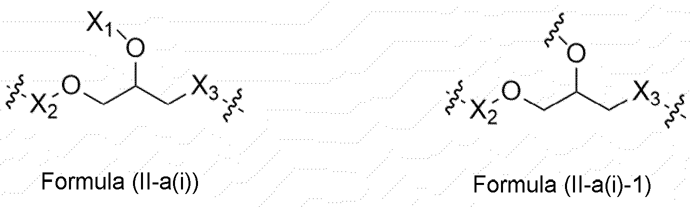 wherein,
X₁ is selected from the group consisting of H, R, or -C(=O)R,
X₂ is selected from the group consisting of C₄₋₈ cycloalkyl, C₄₋₈ heterocycloalkyl, C₄₋₈ aryl, and C₄₋₈ heteroaryl, each of which may be optionally substituted with one or more of -OH, -NH₂, -Q, -CQ₃, -OCH₃, wherein each of Q is independently hydrogen, halogen, methyl, phenyl or benzyl,
X₃ can be selected from the group consisting of CR₂, NZ, O, and S,
wherein R is selected from the group consisting of C₁₋₆ alkyl, C₁₋₆ heteroalkyl, C₁₋₆ alkoxy, C₁₋₆ allyl, C₁₋₆ heteroallyl, C₄₋₈ cycloalkyl, C₄₋₈ heterocycloalkyl, C₄₋₈ aryl, and C₄₋₈ heteroaryl, each of which may be optionally substituted with one or more of -OH, -NH₂, -L, -CL₃, -OCH₃, wherein L is a halogen,
wherein NZ represents an amine based crosslinker and/or hardener selected from the group consisting of poly amide resins, poly(oxypropylene)diamine, 3-aminomethyl-3,5,5-trimethylcyclohexylamine, triethylenetetramines, tetraethylene-pentamines, and trimethylolpropane tris[poly(propylene glycol), amine terminated] ether (T-403).

Preferably, in formula (II-a(i) or in formula (ll-a(i)-1), X₂ is a C₄₋₈ aryl, preferably an aromatic C₆ aryl such as benzene, optionally substituted, and X₃ is selected from O and NZ. Preferably in formula (II-a(i), X₁ is H.

In one embodiment of the method disclosed herein the EBPs comprise the chemical motif of formula (III-a(i)): 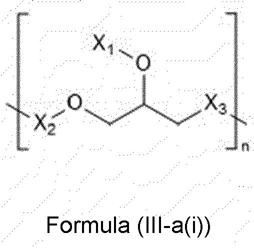 wherein,
X₁ is selected from the group consisting of H, R, or -C(=O)R,
X₂ is selected from the group consisting of C₄₋₈ cycloalkyl, C₄₋₈ heterocycloalkyl, C₄₋₈ aryl, and C₄₋₈ heteroaryl, each of which may be optionally substituted with one or more of -OH, -NH₂, -Q, -CQ₃, -OCH₃, wherein each of Q is independently hydrogen, halogen, methyl, phenyl or benzyl,
X₃ can be selected from the group consisting of CR₂, NZ, O, and S,
wherein R is selected from the group consisting of C₁₋₆ alkyl, C₁₋₆ heteroalkyl, C₁₋₆ alkoxy, C₁₋₆ allyl, C₁₋₆ heteroallyl, C₄₋₈ cycloalkyl, C₄₋₈ heterocycloalkyl, C₄₋₈ aryl, and C₄₋₈ heteroaryl, each of which may be optionally substituted with one or more of -OH, -NH₂, -L, -CL₃, -OCH₃, wherein L is a halogen,
wherein NZ represents an amine based crosslinker and/or hardener selected from the group consisting of poly amide resins, poly(oxypropylene)diamine, 3-aminomethyl-3,5,5-trimethylcyclohexylamine, triethylenetetramines, tetraethylene-pentamines, and trimethylolpropane tris[poly(propylene glycol), amine terminated] ether (T-403), and wherein n represents the motif as repeating, and may be an integer larger than 1.

Preferably, X₁ in formula (III-a(i)) is H, X₂ is a C₄₋₈ aryl, preferably an aromatic C₆ aryl such as benzene, optionally substituted, and X₃ is selected from O and NZ.

In one embodiment of the method disclosed herein the EBPs comprise the chemical motif of formula (II-a(i)-2), formula (II-a(i)-3 or formula (II-a(i)-4): 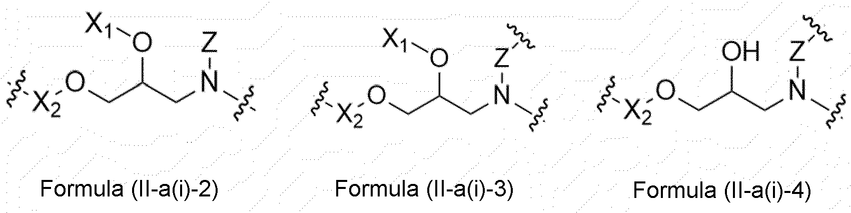 wherein
X₁ is selected from the group consisting of H, R, or -C(=O)R,
X₂ is selected from the group consisting of C₄₋₈ cycloalkyl, C₄₋₈ heterocycloalkyl, C₄₋₈ aryl, and C₄₋₈ heteroaryl, each of which may be optionally substituted with one or more of -OH, -NH₂, -Q, -CQ₃, -OCH₃, wherein each of Q is independently hydrogen, halogen, methyl, phenyl or benzyl,
X₃ can be selected from the group consisting of CR₂, NZ, O, and S,
wherein R is selected from the group consisting of C₁₋₆ alkyl, C₁₋₆ heteroalkyl, C₁₋₆ alkoxy, C₁₋₆ allyl, C₁₋₆ heteroallyl, C₄₋₈ cycloalkyl, C₄₋₈ heterocycloalkyl, C₄₋₈ aryl, and C₄₋₈ heteroaryl, each of which may be optionally substituted with one or more of -OH, -NH₂, -L, -CL₃, -OCH₃, wherein L is a halogen,
wherein NZ represents an amine based crosslinker and/or hardener selected from the group consisting of poly amide resins, poly(oxypropylene)diamine, 3-aminomethyl-3,5,5-trimethylcyclohexylamine, triethylenetetramines, tetraethylene-pentamines, and trimethylolpropane tris[poly(propylene glycol), amine terminated] ether (T-403).

In another embodiment of the method disclosed herein the EBPs comprise the chemical motif of formula (III-b): 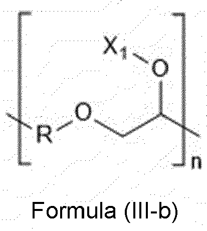 wherein,
R represents phenyl or a derivative thereof, such as bisphenol or a derivative thereof, X₁ represents H, R₁, or -C(=O)R₁, wherein R₁ can be C₁₋₆ alkyl, C₁₋₆ heteroalkyl, C₁₋₆ alkoxy, C₁₋₆ allyl, C₁₋₆ heteroallyl, C₄₋₈ cycloalkyl, C₄₋₈ heterocycloalkyl, C₄₋₈ aryl, and C₄₋₈ heteroaryl, each of which may be optionally substituted with one or more of -OH, -NH₂, -L, -CL₃, -OCH₃, -OCL₃, wherein L is a halogen, and
n represents the motif as repeating, and may be an integer larger than 1.

Preferably, X₁ in formula (III-b) is H.

In one embodiment of the present disclosure, n as defined in Formula (III-b) is an integer of at least 1, such as at least 2, such as at least 5, such as at least 10, such as at least 50, such as at least 100, such as at least 200, such as at least 500, such as at least 1.000, such as at least 10.000.

In one embodiment of the present disclosure, n as defined in Formula (III-b) is an integer between 1 and 1000, such as between 1 and 500, such as between 1 and 400, such as between 1 and 300, such as between 1 and 200, such as between 1 and 100.

In one embodiment of the present disclosure, n as defined in Formula (III-b) is an integer between 50 and 100, such as between 100 and 200, such as between 200 and 300, such as between 300 and 500, such as between 500 and 700, such as between 700 and 1000.

In one embodiment of the present disclosure, R of formula (III-b) is bisphenol or a derivative thereof and may be of the general formula (IV): 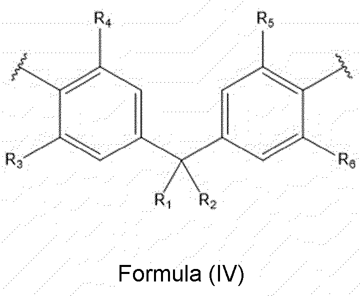 wherein, each of R₁, R₂, R₃, R₄, R₅, and R₆ may independently be selected from -H, -F, - Cl, -Br, -OH, -CH₃, -CF₃, -OCF₃, phenyl, pyridyl, -CN, -NO₂, C₁₋₆ alkyl, and wherein R₁ and R₂ may alternatively come together to form a cyclohexyl moiety optionally substituted with one or more selected from -CH₃, and -CF₃, and wherein the broken bonds of Formula (IV) represents the position of the phenolic oxygens of bisphenol.

Preferably, each of R₁, R₂, R₃, R₄, R₅ and R₆ of Formula (IV) are independently selected from -H, -F, -Cl, -Br, -CH₃, -CF₃ or phenyl.

In another embodiment of the method disclosed herein the EBPs comprise the chemical motif of formula (III-ca): 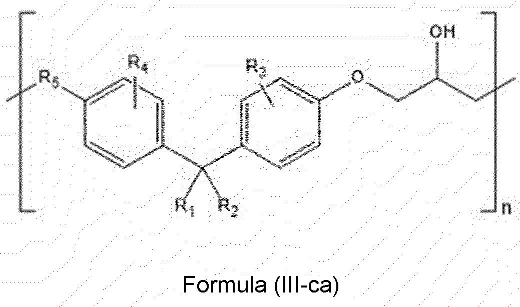 wherein the motif of Formula (III-ca) may comprise from 1 to 4 of each of R₃ and R₄ , and wherein R₁, R₂, R₃, and R₄ are each individually selected from the group consisting of H, C₁₋₆ alkyl, C₁₋₆ alkenyl, CF₃, F, Cl, Br, OH, NO₂, NH₂, phenyl, or wherein R₁ and R₂ may come together to form a C₆-cycloalkyl, and wherein R₅ is selected as O or NZ, wherein NZ represents an amine based crosslinker and/or hardener such as selected from the group consisting of poly amide resins, poly(oxypropylene)diamine, 3-aminomethyl-3,5,5-trimethylcyclohexylamine, triethylenetetramines, tetraethylene-pentamines, and trimethylolpropane tris[poly(propylene glycol), amine terminated] ether (T-403).

In one embodiment, the amine-based crosslinker and/or hardener of formula (III-ca) may be further connected to another bisphenol entity of another epoxy polymer chain, thereby resulting in extensive crosslinking.

In another embodiment of the method disclosed herein the EBPs comprise the chemical motif of formula (III-cb): 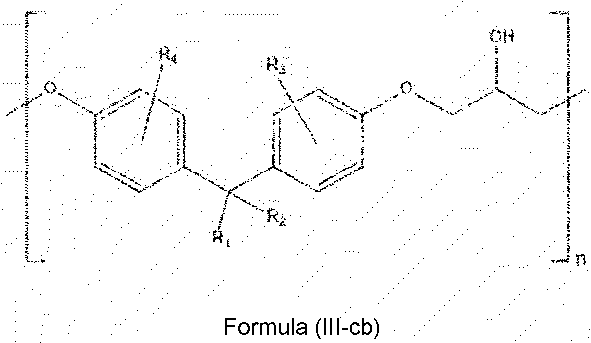 wherein the motif of Formula (III-cb) may comprise from 1 to 4 of each of R₃ and R₄ , and wherein R₁, R₂, R₃, and R₄ are each individually selected from the group consisting of H, C₁₋₆ alkyl, C₁₋₆ alkenyl, CF₃, F, Cl, Br, OH, NO₂, NH₂, phenyl, or wherein R₁ and R₂ may come together to form a C₆-cycloalkyl.

In another embodiment of the method disclosed herein the EBPs comprise the chemical motif of formula (lll-cc): 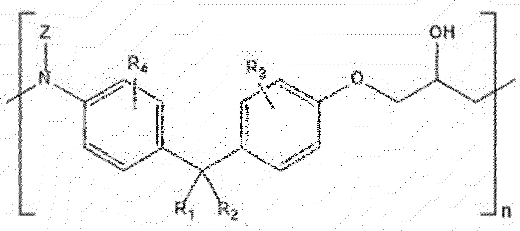 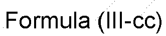 wherein the motif of Formula (III-cc) may comprise from 1 to 4 of each of R₃ and R₄ , and wherein R₁, R₂, R₃, and R₄ are each individually selected from the group consisting of H, C₁₋₆ alkyl, C₁₋₆ alkenyl, CF₃, F, Cl, Br, OH, NO₂, NH₂, phenyl, or wherein R₁ and R₂ may come together to form a C₆-cycloalkyl, and wherein N-Z represents an amine based crosslinker and/or hardener selected from the group consisting of poly amide resins, poly(oxypropylene)diamine, 3-aminomethyl-3,5,5-trimethylcyclohexylamine, triethylenetetramines, tetraethylene-pentamines, and trimethylolpropane tris[poly(propylene glycol), amine terminated] ether (T-403).

In one embodiment of the present disclosure, n as defined in Formulas (III-ca), Formulas (III-cb), and Formulas (III-cc) is an integer of at least 1, such as at least 2, such as at least 5, such as at least 10, such as at least 50, such as at least 100, such as at least 200, such as at least 500.

In one embodiment of the present disclosure, n as defined in Formula (III-ca), Formulas (III-cb), and Formulas (III-cc) is an integer between 1 and 1000, such as between 1 and 500, such as between 1 and 400, such as between 1 and 300, such as between 1 and 200, such as between 1 and 100.

In one embodiment of the present disclosure, n as defined in Formula (III-ca), Formulas (III-cb), and Formulas (III-cc) is an integer between 50 and 100, such as between 100 and 200, such as between 200 and 300, such as between 300 and 500, such as between 500 and 700, such as between 700 and 1000.

In another embodiment of the method disclosed herein the EBPs comprise the chemical motif of formula (III-da): 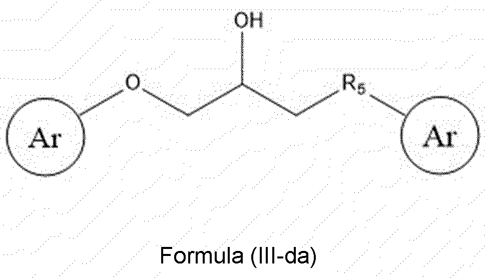 wherein R₅ is selected as O or NZ, wherein NZ represents an amine based crosslinker and/or hardener selected from the group consisting of poly amide resins, poly(oxypropylene)diamine, 3-aminomethyl-3,5,5-trimethylcyclohexylamine, triethylenetetramines, tetraethylene-pentamines, and trimethylolpropane tris[poly(propylene glycol), amine terminated] ether (T-403), and wherein Ar represent an aromatic ring such as phenyl or naphthyl, preferably phenyl. The aromatic ring of formula (III-da) may be substituted or cross-linked in the polymer, such as representing yet another bisphenol or bisphenol diglycidyl ether derived moiety.

In another embodiment of the method disclosed herein the EBPs comprise the chemical motif of formula (III-db): 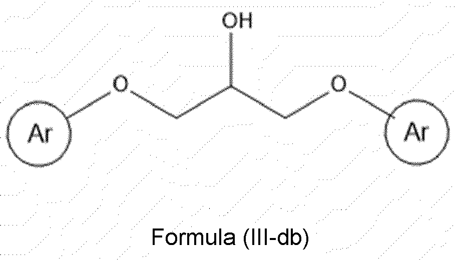 wherein Ar represent an aromatic ring such as phenyl or naphthyl, preferably phenyl. The aromatic ring of formula (III-db) may be substituted or cross-linked in the polymer such as representing yet another bisphenol or bisphenol diglycidyl ether derived moiety.

In another embodiment of the method disclosed herein the EBPs comprise the chemical motif of formula (III-dc): 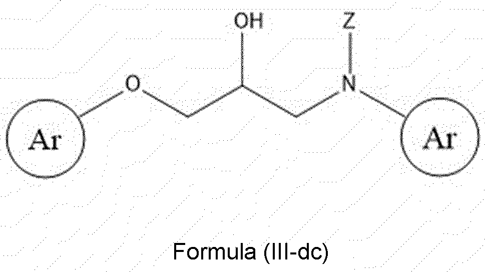 wherein N-Z represents an amine based crosslinker and/or hardener selected from the group consisting of poly amide resins, poly(oxypropylene)diamine, 3-aminomethyl-3,5,5-trimethylcyclohexylamine, triethylenetetramines, tetraethylene-pentamines, and trimethylolpropane tris[poly(propylene glycol), amine terminated] ether (T-403), and wherein Ar represent an aromatic ring such as phenyl or naphthyl, preferably phenyl. The aromatic ring of formula (III-dc) may be substituted or cross-linked in the polymer, such as representing yet another bisphenol or bisphenol diglycidyl ether derived moiety.

In one embodiment of the present disclosure, the EBPs deconvoluted in the method comprise one or more cross-linkers. In one embodiment disclosed herein, the cross-linkers are selected from the group of poly amide resins, poly(oxypropylene)diamine, 3-aminomethyl-3,5,5-trimethylcyclohexylamine, triethylenetetramines, tetraethylene-pentamines and trimethylolpropane tris[poly(propylene glycol), amine terminated] ether (T-403).

In one embodiment of the present disclosure, the cross-linkers may be selected from conventionally used epoxy-hardeners known to a person of skill in the art.

In one embodiment of the present disclosure, the EBPs comprise one or more of epoxy-hardeners selected from the group of poly amide resins, poly(oxypropylene)diamine, 3-aminomethyl-3,5,5-trimethylcyclohexylamine, triethylentetramines, tetraethylene-pentamines and trimethylolpropane tris[poly(propylene glycol), amine terminated] ether (T-403).

In one embodiment of the method herein disclosed, the monomer comprised in the EBPs is selected from the group consisting of Bisphenol A, Bisphenol AP, Bisphenol AF, Bisphenol B, Bisphenol BP, Bisphenol C, Bisphenol E, Bisphenol F, Bisphenol G, Bisphenol M, Bisphenol S, Bisphenol P, Bisphenol PH, Bisphenol TMC, Bisphenol Z, and derivatives thereof.

In one embodiment of the present disclosure, the monomers in the EBPs comprise bisphenol A or a derivative thereof.

In one embodiment of the present disclosure, the EBPs are selected from the group consisting of Bisphenol A based EBPs, Bisphenol AP based EBPs, Bisphenol AF based EBPs, Bisphenol B based EBPs, Bisphenol BP based EBPs, Bisphenol C based EBPs, Bisphenol C based EBPs, Bisphenol E based EBPs, Bisphenol F based EBPs, Bisphenol G based EBPs, Bisphenol M based EBPs, Bisphenol S based EBPs, Bisphenol P based EBPs, Bisphenol PH based EBPs, Bisphenol TMC based EBPs, Bisphenol Z based EBPs. In another embodiment, the EBPs are bisphenol A based EBPs.

The EBPs employed in the methods of the present disclosure are generally not soluble in any conventional solvents. Therefore, the base-mediated deconvolution reaction is limited to surface reactivity which can be time consuming. For this reason, the EBPs may be shredder, grinded, filed or by similar means turned into a powder prior to performing the method, such as by use of a flat file, to achieve a larger surface area. Therefore, in one embodiment of the present disclosure, the EBP is a powder or granulate. Powders or granulates of polymers may be characterized by standard techniques known to persons of ordinary skill in the field of physical sciences, such as e.g., dynamic light scattering according to ISO13320:2020, and expressed in numerical values such as D₅₀ and D₉₀ (or another number between 1 and 100). As such, the value of Dₙ refers the particle size of which *n* % of the measured sample is smaller.

In one embodiment of the present disclosure, the EBP powder size distribution is characterized by a D₉₀ of less than 1.000 µm, such as less than 900 µm, such as less than 800 µm, such as less than 700 µm, such as less than 600 µm, such as less than 500 µm, such as less than 400 µm, such as less than 300 µm, such as less than 200 µm, such as less than 100 µm.

In one embodiment of the present disclosure, the EBP powder size distribution is characterized by a D₉₀ of less than 500 µm, such as less than 400 µm.

In one embodiment of the present disclosure, the EBP powder size distribution is characterized by a D₉₀ from 100 µm to 1000 µm, such as from 100 µm to 200 µm, such as from 200 µm to 275 µm, such as from 275 µm to 325 µm, such as from 325 µm to 350 µm, such as from 350 µm to 375 µm, such as from 375 µm to 400 µm, such as from 400 µm to 425 µm, such as from 425 µm to 500 µm, such as from 500 µm to 600 µm, such as from 600 µm to 700 µm, such as from 700 µm to 800 µm, such as from 800 µm to 1.000 µm
In one embodiment of the present disclosure, the EBP powder size distribution is characterized by a D₉₀ from 200 µm to 500 µm, such as from 300 µm to 400 µm.

In one embodiment of the present disclosure, the EBP powder size distribution is characterized by a D₅₀ of less than 300 µm, such as less than 250 µm, such as less than 225 µm, such as less than 200 µm, such as less than 175 µm, such as less than 150 µm.

In one embodiment of the present disclosure, the EBP powder size distribution is characterized by a D₅₀ of less than 225 µm, such as less than 175 µm.

In one embodiment of the present disclosure, the EBP powder size distribution is characterized by a D₅₀ from 50 µm to 300 µm, such as from 50 µm to 75 µm, such as from 75 µm to 100 µm, such as from 100 µm to 125 µm, such as from 125 µm to 150 µm, such as from 150 µm to 175 µm, such as from 175 µm to 250 µm, such as from 250 µm to 300 µm.

In one embodiment of the present disclosure, wherein the EBP powder size distribution is characterized by a D₅₀ from 75 µm to 200 µm, such as from 100 µm to 175 µm.

In one embodiment of the present disclosure, the method as described herein is conducted in a closed vessel, such as in a closed vessel under autogenous pressure. By "autogenous pressure" is meant the pressure that is ambient or otherwise created inside a closed vessel during a reaction, such as when a reaction is heated or gasses are produced within the closed vessel. In other embodiments, the method as described herein may be conducted in an open container or vessel in direct contact with the atmosphere.

In one embodiment of the present disclosure, the autogenous pressure is between/at least 0.10 MPa to 1.00 MPa (14.5 to 145 psi).

In one embodiment of the present disclosure, the method as described herein is performed in an inert atmosphere, such as an inert atmosphere consisting essentially of nitrogen (N₂) or Argon (Ar). In another embodiment, the method is performed in a non-inert atmosphere, such as using ambient air.

In order to increase the conversion rate of the reaction to a point where is has practical use, the suspension comprising base, solvent and EBP polymer is heated to a temperature of at least 130°C for at least 2 hours. In one embodiment of the present disclosure, the suspension is heated to a temperature of at least 130 °C, such as at least 140 °C, such as at least 150 °C, such as at least 160 °C, such as at least 170 °C, such as at least 180 °C, such as at least 190 °C, such as at least 200 °C, such as at least 210 °C, such as at least 220 °C, such as at least 230 °C, such as at least 240 °C, such as at least 250 °C, for at least 2 hours. Preferably the suspension is heated to at least 130 °C, more preferably to at least 170 °C, most preferably to at least 190 °C, for at least 2 hours.

In another embodiment of the present disclosure, the suspension is heated to a temperature between 130 °C and 250 °C, such as between 130 °C and 150 °C, such as between 150 °C and 170 °C, such as between 170 °C and 190 °C, such as between 190 °C and 210 °C, such as between 210 °C and 230 °C, such as between 230 °C and 250 °C for at least 2 hours. Preferably the suspension is heated to a temperature between 130 °C and 230 °C, more preferably between 170 °C and 210 °C, for at least 2 hours.

In one embodiment of the present disclosure, the suspension is heated for at least 4 hours, such as for at least 8 hours, such as for at least 16 hours, such as for at least 24 hours.

In one embodiment of the present disclosure, the suspension is heated for a time ranging from 2 hours to 10 days, such as from 2 hours to 4 hours, such as from 4 hours to 8 hours, such as from 8 hours to 16 hours, such as from 16 hours to 24 hours, such as from 24 hours to 2 days, such as from 2 days to 3 days, such as from 3 days to 5 days, such as from 5 days to 10 days. Preferably the suspension is heated from 16 hours to 3 days, more preferably from 16 hours to 2 days, most preferably from 24 hours to 2 days.

It is within the scope of the present disclosure to provide methods that allow for deconvolution of EBPs cured for themselves or as matrix for fibres, such as to create FREBP composites. This makes the present invention attractive from the stand-point of circular economy. Traditionally investigated methodologies focusing on the recovery of fibres rely on pyrolysis, electric discharge or supercritical solvents. These harsh conditions are impractical for industrial volumes. Furthermore, they destroy rather than deconstruct the polymer matrix, usually and end up damaging the fibres. Chemical deconstruction approaches are strongly underdeveloped, due to the immense challenge that originates in the physical and chemical stability of cured epoxy resins, however has in recent years shown that some FREBPs can be broken apart by use of acetic acid to recover the fibres.

To demonstrate that the claimed method of the present disclosure can also be used to deconstruct resins from fiber-reinforced composites, the method needs to tolerate residues from treatments that can be used to separate fibers from the resin. It is known in the art that acetic acid can be used to swell cured epoxy resins in order to allow more efficient catalytic deconstruction. It is therefore relevant to demonstrate that the method of the present disclosure is also applicable to EPBs that have been subjected to a step of swelling using acetic acid. However in one embodiment of the present disclosure, the EBPs have not been subjected to a step of swelling using acetic acid prior to the step of contacting said EBPs with the suspension comprising at least one aprotic apolar organic solvent and an oxygen-containing base.

In one embodiment of the present disclosure, the epoxy-based polymer may be mixed with fibres, such as in fibre-reinforced composites, in particular those where the fibres are glass fibre or carbon fibre.

In one embodiment of the method herein disclosed, the fibres in the FREBPs are selected from a group consisting of glass fibres, carbon fibres, cellulose, lignin, aramid, and asbestos. In one embodiment of the present disclosure, the fibres in the FREBPs are glass fibres and/or carbon fibres.

In one embodiment of the present disclosure, the method as described herein further comprising a step of pre-treatment of the epoxy-based polymer, or when the EBP is in the presence of fibres in a FREBP composite, may further comprise a step of pre-treatment of the composite for the purpose of fibre separation.

In one embodiment of the present disclosure, the pre-treatment step comprises contacting the epoxy-based polymer or a composite thereof with a swelling agent.

The method of the present disclosure takes place in a solvent comprising at least one aprotic apolar organic solvent, preferably an organic solvent with a high boiling point, such as a boiling point above 100 °C. It is also preferred within the method of the present disclosure that the solvent is a non-coordinating solvent, such as solvents known to a person of ordinary skill in the art of chemical synthesis.

Thus, in the present disclosure, the solvent is an aprotic apolar organic solvent, and is preferably a non-coordinating apolar organic solvent. In this context apolar is considered equivalent to non-polar. The distinguishing between polar and non-polar solvents is well-known to those skilled in the art. Solvents composed of hydrocarbons such as heptane, toluene, cymene and xylenes are generally accepted as being nonpolar. Such terminology is also known to persons of ordinary skill, and is widely used and accepted in the field of chemical synthesis.

In one embodiment of the present disclosure, the aprotic apolar organic solvent is a solvent comprising only carbon and hydrogen atoms. In a further embodiment, the organic solvent is selected from the group consisting of toluene, benzene, xylene, mesitylene, cumene, cymene, and xylol, or a structural isomer of any one of these, such as *ortho-, meta-,* and *para-* isomers known to persons of ordinary skill.

In one embodiment of the present disclosure, the organic solvent is selected from the group consisting of toluene, xylene and cymene, or a structural isomer of any one of these.

In one embodiment of the present disclosure, the organic solvent is selected from the group consisting of benzene, toluene, cymenes, and xylenes, such as p-cymene and o-xylene.

The method of the present disclosure makes use of at least one oxygen-containing base in the base-mediated deconstruction of epoxy-based polymers. In one embodiment of the present disclosure, the oxygen-containing base is a nucleophilic base. In one embodiment of the present disclosure, the oxygen-containing base is a non-nucleophilic base.

In one embodiment of the present disclosure, the base is a hydroxide selected from the group consisting of NaOH, LiOH, KOH, CsOH, RbOH, Ca(OH)₂, Sr(OH)₂, and Ba(OH)₂; or an alkoxide selected from the group consisting of butoxide, tert-butoxide, methoxide, ethoxides, and isopropoxide, including sodium or potassium salts of these.

In one embodiment of the present disclosure, the base is selected from the group consisting of NaOH, KOH, NaOBu, and NaOtBu, preferably NaOH or KOH.

In one embodiment of the method, the EBPs have not been chemically treated prior to the step of contacting said EBPs with the suspension comprising at least one aprotic apolar organic solvent and an oxygen-containing base

In one embodiment of the present disclosure, the method does not comprise or make use of one or both of hydrogen peroxide and/or ascorbic acid, such as does not comprise or make use of hydrogen peroxide, or does not comprise or make use of ascorbic acid.

Due to the complex and varying nature of cross-linked and/or cured epoxy polymer resins, it is difficult if not impossible to know the exact amount of targeted C-O and/or C-N linkages in the resin, and therefore also to add the base in equivalent amounts. Therefore, the base is added in a weight percentage (wt%) of the EBP weight.

In one embodiment of the present disclosure, the base is added in at least 10 wt% by weight of the EBP, such as at least 20 wt%, such as at least 30 wt%, such as at least 40 wt%, such as at least 50 wt%, such as at least 60 wt%, such as at least 70 wt%, such as at least 100 wt%, such as at least 150 wt%, such as at least 200 wt%, such as at least 300 wt%, such as at least 400 wt% by weight of the EBP.

In another embodiment of the present disclosure, the base is added in a range from 10 wt% to 100 wt% by weight of the EBP, such as from 10 wt% to 20 wt%, such as from 20 wt% to 30 wt%, such as from 30 wt% to 40 wt%, such as from 40 wt% to 45 wt%, such as from 45 wt% to 50 wt%, such as from 50 wt% to 55 wt%, such as from 55 wt% to 60 wt%, such as from 60 wt% to 70 wt%, such as from 70 wt% to 80 wt%, such as from 80 wt% to 90 wt%, such as from 90 wt% to 100 wt% by weight of the EBP.

In one embodiment, the base is added in a range from 40 wt% to 60 wt% by weight of the EBP, preferably 50 wt%.

### Examples

Information in the Examples, such as controls, or methods falling outside the scope of the claims is provided for illustrative purposes only.

### General Information

Unless otherwise stated, all reactions were set-up and worked up in a glovebox under an atmosphere of argon. All chemicals were purchased from Sigma-Aldrich, Tokyo Chemical Industry (TCI), VWR Chemicals or Strem Chemicals and used as received unless stated otherwise. NaOH (98.6% purity) was purchase from VWR Chemicals as pellets and crushed in a mortar, yielding a powder. THF, toluene, CH₂Cl₂ and MeCN were retrieved from a MBraun SP-800 purification system, degassed using argon and stored over 3 Å molecular sieves. The remaining solvents were purchased from Sigma-Aldrich degassed using argon, stored over 3 Å molecular sieves and used without further purification.

**Thin layer chromatography** (TLC) was carried out on pre-coated aluminium sheets ALUGRAM^{®} Xtra SIL G/UV254 purchased by Macherey-Nagel. Visualisation of the products was achieved by UV-light irradiation (366 nm) and / or staining with a potassium permanganate in water.

**Flash column chromatography** was carried out using Silica gel (0.040 - 0.063 mm/ 230 - 400 mesh) ASTM purchased from Macherey-Nagel. Automated flash column chromatography (AFCC) was carried out with Interchim PuriFlash XS520Plus with 30 µm prepacked columns. Celite^{®}545, coarse, was used for filtration.

**Gas chromatography** - **mass spectrometry** (GC-MS) were measured with an Agilent 8890 gas chromatograph coupled with an Agilent 5977B mass selective detector.

**High resolution mass spectrometry** (HRMS): ESI(+) spectral analysis were measured with a Bruker Maxis Impact Spectrometer. MALDI spectral analysis were measured on a Bruker Autoflex maX MALDI-TOF MS spectrometer using a MTP 384 target plate polished steel BC.

**NMR spectra:** ¹H NMR, ¹³C NMR and ³¹P NMR spectra were recorded on a Bruker 400 MHz Ascend spectrometers at 25 °C unless otherwise specified. Chemical shifts were given as δ value (ppm) with reference to residual solvent signal of the deuterated solvent. The peak patterns are indicated as follows: s, singlet; d, doublet; t, triplet; m, multiplet; q, quartet. Multiplicities reported for ¹³C NMR spectra were assigned using DEPT-90 and/or DEPT-135 spectra. The coupling constants, J, are reported in Hertz (Hz). The spectra were calibrated to the residual solvent signals. NMR spectra were processed with MestReNova Version 14.2.1-27684.

**Particle size distributions** were determined using a Malvern Mastersizer 2000 instrument with a Hydro S dispersion unit (Malvern Panalytical). The measurements were performed by means of dynamic light scattering and particles in the size interval from 0.02-2.000 µm were measured. The sample was measured with constant stirring to avoid sedimentation according to ISO13320:2020 using a stirring rate of 3500 rpm and laser wavelengths of 633 nm and 466 nm. A refractive index of 1.5 and an absorption of 0.1 was assumed for the size distribution modelling of a sample of spherical particles. The result is reported as an average of triplicate measurement.

### Example 1 - Preparation of epoxy model

### Step 1. Preparation of Me-BPA (4-(2-(4-methoxyphenyl)propan-2-yl)phenol)

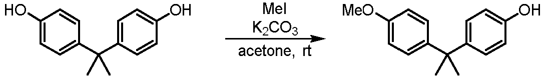

To a 250 mL round bottom flask equipped with stir bar and a suspension of K₂CO₃ (10.4 g, 75.0 mmol) in 100 mL acetone, bisphenol A (11.4 g, 50.0 mmol) was added while stirring under air at room temperature. Then methyl iodide (7.1 g, 50.0 mmol) was added dropwise via syringe and the mixture was stirred at room temperature overnight. Afterwards, the reaction mixture was filtered and the organic phase was concentrated under *vacuo.* The mixture was then subject to flash column chromatography
with silica gel using a gradient of 15/1 pentane/ethyl acetate to 10/1 pentane/ethyl acetate afforded Me-BPA as colourless highly viscous oil in a yield of 37% (4.50 g, 18.6 mmol).

¹H NMR (CDCl₃, 400 MHz, 25 °C): δ = 7.16 -7.12 (m, 2H), 7.11 - 7.08 (m, 2H), 6.82 - 6.78 (m, 2H), 6.74- 6.71 (m, 2H), 4.54 (s, 1H), 3.78 (s, 3H), 1.64 (s, 6H) ppm.

### Step 2. Preparation of epoxy model (1,3-bis(4-(2-(4-methoxyphenyl)propan-2-yl)phenoxy)propan-2-ol)

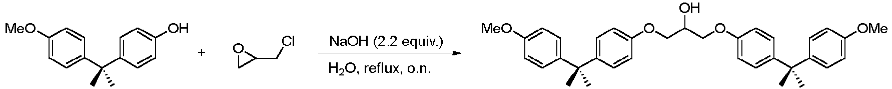

To a 100 mL flask equipped with stir bar and a mixture of Me-BPA (2.42 g, 10.0 mmol) and 50 mL H₂O was added NaOH (0.44 g, 11 mmol) and the mixture was stirred for 10 min. Then, epichlorohydrin (0.46 g, 5 mmol) was added and the mixture was heated to reflux overnight. Afterwards the reaction mixture was cooled down to room temperature and extracted with DCM (30 mL x 3). The organic phase was dried over Na₂SO₄ and concentrated under vacuo. Then the mixture was purified by flash column chromatography with a gradient of 10/1 pentane/ethyl acetate to 5/1 pentane/ethyl acetate, afforded model 1 as colorless highly viscous oil. In addition, the solvent residual was removed under *vacuo* with heating to afford desired product in a yield of 50 % (1.37 g, 2.5 mmol).

¹H NMR (CDCl₃, 400 MHz, 25 °C): δ = 7.16 - 7.12 (m, 8H), 6.84 - 6.78 (m, 8H), 4.36 (h, *J* = 5.3 Hz, 1H), 4.15 - 4.09 (m, 4H), 3.78 (s, 6H), 2.54 (d, *J* = 4.0 Hz, 1H), 1.64 (s, 12H) ppm; ¹³C NMR (CDCl₃, 101 MHz, 25 °C): δ = 157.5 (s, 2C), 156.3 (s, 2C), 143.9 (s, 4C), 143.1 (s, 2C), 127.9 (s,4C), 127.8 (d, 4C), 114.0 (d, 4C), 113.4 (d, 4C), 68.9 (d, 1C), 68.7 (t, 2C), 55.3 (q, 2C), 41.8 (s, 2C), 31.2 (q, 4C) ppm; HRMS (ESI+): calculated [M+Na]⁺ = [C₃₅H₄₀O₅+Na]⁺ 563.2768; found 563.2762.

### Example 2 - proof of concept by degradation of epoxy model using only base in the absence of metal catalyst

The use of metal catalysts for industrial scale applications, especially those catalysts based on metals such as ruthenium, palladium, rhodium, platinum and gold is highly undesirable as it often renders an otherwise useful reaction unfeasible from an financial point of view. To this end, it was decided to attempt epoxy model deconstruction in the absence of any metal catalysts, employing only base and a non-coordinating solvent such as toluene at elevated temperature and pressure for the deconstruction. The following procedure was used.

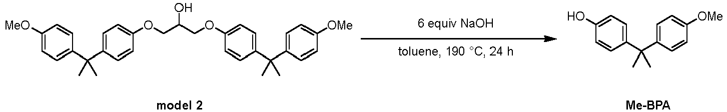

1 equivalent (0.1 mmol) of epoxy model substrate was loaded into 10 ml COtube. Under an atmosphere of air, 2 ml of toluene and 6 equivalents (0.6 mmol) of NaOH were added and the reaction vessel sealed. The use of 6 equivalents NaOH was decided based on previous unpublished data demonstrating that 6 equivalents provides the optimum yield for the otherwise same reaction conditions, and fewer equivalents resulted in lower isolated yields. The reaction mixture was then stirred at 650 rpm in an aluminium block at 190 °C for 24 h. The reaction mixture was then cooled to room temperature, quenched using 2 ml of 4 M aqueous HCl solution and extracted using ethyl acetate. After this, *either* (for isolated yields) the solvent removed *in vacuo* and products isolated using column chromatography or 1,3,5-trimethoxybenzene as internal standard was added and ¹H NMR spectra in CDCl3 was taken in order to determine the yield without isolation, to account for any mechanical loss.

**Table 1 - conversion of epoxy model and Bisphenol A yield obtained in Example 2.**

| **Entry** | **Variation** | **Conversion (%)** | **Yield (%)** |
|---|---|---|---|
| **1** | none | 60^{a)} | 33^{a)} |

| | | | |
|---|---|---|---|
| ^{a)} as determined by ¹H-NMR. 0.1 mmol 1,3,5-trimethoxybenzene was used as internal standard to have ¹H NMR to confirm the conversion (leftover of substrate) and yield. | | | |

### Example 3 - Screening of solvent, reaction temperature and time for base-mediated deconstruction of commercially available epoxy resin

With the encouraging proof-of-concept result obtained in Example 2 in hand, showing that deconstruction of epoxy model systems was possible using only base as a mediator, the same reaction with several modifications were examined on the realworld epoxy resin Airstone^{™} 760E/766H to optimized reaction parameters.

### Sample preparation

As the cross-linked resins tested in the present and following examples are not soluble in the tested solvents, degradation into base constituents via chemical reactions is limited to reactions taking place at the surface of the resin. Therefore, to maximize surface area and reduce reaction time, a block of the cured resin was filed into a powder using a flat file as a tool. The cured resin Airstone^{™} 760E/766H (but also UHU plus endfest 2-component glue, Roizefar epoxy resin, and Sicomin SR infugreen 810/SD8822 in Example 4) was thus filed into powders. The powder size of Airstone^{™} 760E/766H was determined using dynamic light scattering (Fig. 4) and powders of the remaining resins were assumed to be of similar size since the same tool and procedure was used.

Dynamic light scattering measurements of Airstone^{™} 760E/766H powder revealed that 10% of the particles have a diameter below 32.8 µm (D10 value), 50% are above and 50% below 134.1 µm in diameter (D50 value) and 90% have a diameter below 380.5 µm (D90 value).

Due to the relatively high pressure that toluene will build up at 190 °C, similar aprotic and non-coordinating solvents with higher boiling points were considered (Table 2). Both o-xylol (bp 144 °C) and p-cymene (bp 177 °C) gave comparable yields of 75% of recovered BPA each after 24 h (entry 2 and 3). *p*-Cymene is especially interesting, as this nontoxic solvent can be sourced from biomass. No significant difference in yield was observed as a consequence of different boiling points, as long as the boiling point is above 100 °C.

The method demonstrates a clear temperature dependence evidenced by the BPA yield dropping from 79 % at 190 °C to 59 % at 170 °C and further decreases until no BPA could be detected when the reaction was run at 110 °C (entries 1 and 4-7).

Extending the reaction time to 2 days gave no significant improvement over the reaction time of 24 h (entry 8). We conclude that 81% is the maximal amount of BPA that can be recovered from Airstone^{™} 760E/766H using this methodology. The remaining BPA might be adjacent to crosslinking motifs that are more challenging to cleave.

**Table 2 - screening of solvents, temperature and reaction time for optimized base-mediated deconstruction of epoxy resins, exemplified by Airstone^{™} 760E/766H**

| 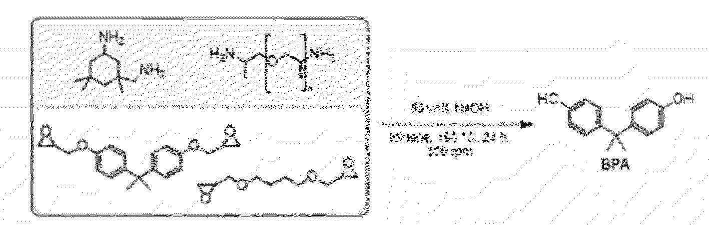 | | |
|---|---|---|
| **entry** | **variation** | **yield BPA^{a)}** |
| **1** | none | 79 % (34.1 mg) |
| **2** | *o*-xylol instead of toluene | 75 % (32.1 mg) |
| **3** | *p*-cymene instead of toluene | 75 % (32.5 mg) |
| **4** | Temperature, 170 °C | 59 % (25.5 mg) |
| **5** | Temperature, 150 °C | 27 % (12.0 mg) |
| **6** | Temperature, 130 °C | 14 % (6.0 mg) |
| **7** | Temperature, 110 °C | 0 % (0 mg) |
| **8** | Reaction time increased to 2 days | 81 % (34.5 mg) |
| **9** | 500 mg scale, o-xylol, 3 d | 74 % (158 mg) |
| **10** | No NaOH | 0% |
| **11** | 25 wt% NaOH | 33 % (14.3 mg) |
| **12** | KOH instead of NaOH | 61 % (26.4 mg) |
| **13** | 5 vol% H₂O | 0% |
| **14** | 12 vol% MeOH, 170 °C | 45 % (19.1 mg) |
| **15** | 10 mol% TBAC^{b)}, 170 °C | 45 % (19.3 mg) |
| **16** | 10 mol% 15-crown-5, 170 °C | 55 % (23.5 mg) |

| | | |
|---|---|---|
| a) Yield of product isolated *via* flash column chromatography. b) TBAC = tetrabutylammonium chloride. | | |

The results can be summarized as showing that the temperature should be as high as possible, preferably at least 190°C for the volumes and reaction vessels specified. The results also demonstrate that no particular solvent is preferred, as long as the solvent is non-protic, non-polar and non-coordinating. In this aspect, toluene, o-xylol and p-cymene seems to be useful to the same degree.

### Example 4 - General procedure for deconvolution of epoxy resins and recovery of Bisphenol A

General procedure: 100 mg of powdered resin were given into 40 ml COtube. Under air, 4.2 ml of toluene and 1.26 mmol of base were added and the reaction vessel sealed. The reaction mixture is stirred at 300 rpm in an aluminium block at 190 °C for 24 h. The reaction mixture is then cooled to room temperature, quenched using 10 ml of 4 M aqueous HCl solution and extracted using ethyl acetate. Afterwards, 1 g of Celite was added to the mixture and after concentration under *vacuo* the mixture was subject to column chromatography to afford bisphenol A. Column chromatography using a gradient of 6/1 pentane/ethyl acetate to 4/1 pentane/ethyl acetate afforded bisphenol A.

The deconstruction of Airstone^{™} 760E/766H (approx. BPA content 43 wt%, see Fig. 2) was carried out according to the general procedure described above. Airstone^{™} 760E/766H is an infusion resin for wind turbine systems, such as wind turbine blades, which was provided by Olin Corporation^{®} and is prepared from the following reagents: bis-[4-(2,3-epoxipropoxi)phenyl]propane (DGEBA), 1,4-bis(2,3-epoxypropoxy)butane (BDDE), poly(oxypropylene) diamine and 3-aminomethyl-3,5,5-trimethylcyclohexylamine.

According to the available safety data sheets, 760E contains
- 75.0% bis-[4-(2,3-epoxipropoxi)phenyl]propane (DGEBA); and
- < 25.0% 1,4-bis(2,3-epoxypropoxy)butane (BDDE).
while 766H contains
- ≤ 50.0 < 75.0% poly(oxypropylene) diamine; and
- ≤ 25.0 < 50.0% 3-aminomethyl-3,5,5-trimethylcyclohexylamine.

The above general procedure was implemented using 100 mg of the powdered resin, 50.0 mg (1.26 µmol, 50.0 wt%) of NaOH, and 4.2 ml toluene. Column chromatography afforded BPA as a colourless solid and a rest fraction as brown highly viscous oil. Yield of BPA (based on theoretical maximum): 34.7 mg (80.7%).

The deconstruction of UHU^{®} 2-component glue (UHU plus endfest 2-K-Epoxidharzkleber 45670) (approx. BPA content 34 wt%, see Fig. 3a) was carried out according to the general procedure described above. The adhesive was prepared by mixing the two individual components in a 1g to 1g ratio and letting it harden overnight at room temperature.

The above general procedure was implemented using 100 mg of the powdered resin, 50.0 mg (1.26 µmol, 50.0 wt%) of NaOH, and 4.2 ml toluene. Column chromatography afforded BPA as a colourless solid and a rest fraction as brown highly viscous oil. Yield of BPA (based on theoretical maximum): 28.5 mg (83.8%).

The deconstruction of Roizefar^{®} Epoxy Resin (approx. BPA content 30 wt%, see Fig. 3b) was carried out according to the general procedure described above. The twocomponent resin, produced by Shenzhen Fengao Technology Co., Ltd. was prepared by mixing the two individual components in a 1g to 1g ratio and letting it harden overnight at room temperature.

The above general procedure was implemented using 100 mg of the powdered resin, 50.0 mg (1.26 µmol, 50.0 wt%) of NaOH, and 4.2 ml toluene. Column chromatography afforded BPA as a colourless solid and a rest fraction as brown highly viscous oil. Yield of BPA (based on theoretical maximum): 25.9 mg (86.3%). Additionally, 12.2 mg of phenol could be isolated separately.

The deconstruction of Sicomin SR infugreen^{™} 810/SD8822 produced by Sicomin Epoxy Systems (approx. BPA content 36 wt%, see Fig. 3c) was carried out according to the general procedure described above. The resin was prepared by mixing SR infugreen^{™} 810 (100 g) with a hardener SD8822 (32.0 g), which was then degassed for 20 min under vacuum, poured into preheated silicone moulds (50 °C) and cured at 50 °C for 1 h and 80 °C for 3 h to give the hardened resin.

According to the available safety data sheet, SR infugreen^{™} 810 contains:
- 50 ≤ x% < 100 bisphenol-A-(epichlorhydrin); epoxy resin (number average molecular weight ≤ 700);
- 10 ≤ x% < 25 1,4-bis(2,3-epoxypropoxy)butane (BDDE); and
- 2.5 ≤ x% < 10 bisphenol-F-(epichlorhydrin); epoxy resin (number average molecular weight ≤ 700).

While SD8822 contains:
- 50 ≤ x% < 100 3-aminomethyl-3,5,5-trimethylcyclohexylamine;
- 25 ≤ x% < 50 poly(oxypropylene) diamine; and
- 2.5 ≤ x% < 10 trimethylolpropane tris[poly(propylene glycol), amine terminated] ether.

The above general procedure was implemented using 100 mg of the powdered resin, 50.0 mg (1.26 µmol, 50.0 wt%) of NaOH, and 4.2 ml toluene. Column chromatography afforded BPA as a colourless solid and a rest fraction as brown highly viscous oil. Yield of BPA (based on theoretical maximum): 21.0 mg (58.3%).

These results in combination show that the method herein described can be applied to degrade a wide variety of commercially available EBPs, further allowing recovery of BPA. This highlights the vast potential of this methodology in the recycling of such materials.

### Example 5 - scale-up experiment based on cured Airstone^{™} 760E/766H resin

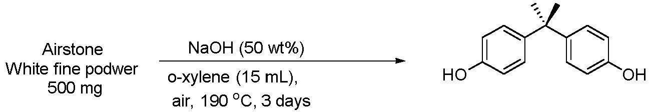

The reaction was set up following the general procedure outlined above under air with powdered Airstone^{™} 760E/766H (500 mg), NaOH (250 mg, 50 wt%), o-xylene (15 mL) in a 40 mL COtube (SyTracks) at 190 °C at 300 rpm. After 3 days, the reaction was cooled down to room temperature and quenched with HCl (4 M, 8 mL). The reaction mixture was extracted with EtOAc (6 mL x 4) and i) separated using automated column chromatography over silica, using a gradient of heptane 100% to ethyl acetate 100%, or ii) dried over with MgSO4 and the solvent was removed under *vacuo.*

Proces i) here above produces BPA as an off white solid in a yield of 158 mg (73%). R_{f} (pentane/ethyl acetate 4/1, silica gel) = 0.21; ¹H NMR (CDCl₃, 400 MHz, 25 °C): δ = 7.11 - 7.07 (m, 4H), 6.76 - 6.70 (m, 4H), 4.57 (s, 2H), 1.62 (s, 6H) ppm.

Process ii) here above affords 229 mg crude BPA as a dark highly viscous oil. The crude BPA was analyzed using ¹H NMR spectroscopy in deuterated acetone, showing acceptable purity for further use in synthesis purposes (Fig. 5). ¹H NMR (CDCl₃, 400 MHz, 25 °C): δ = 7.06 - 7.03 (m, 4H), 6.73 - 6.70 (m, 4H), 2.99 (s, 2H), 1.58 (s, 6H) ppm.

The present example demonstrates that the present method is not limited to small-scale recycling, rather the quantity of resin can as a first approximation be quintupled without any significant adverse effects on the BPA yield.

### Example 6 - deconvolution of resin from fiber-reinforced composites based on cured Airstone^{™} 760E/766H resin

To demonstrate that the claimed method of the present disclosure can also be used to deconstruct resins from fiber-reinforced composites, the method needs to tolerate residues from treatments that can be used to separate fibers from the resin. Acetic acid has been previously reported to physically fragment thermoset epoxy resin. Furthermore, the first commercial blades designed for such a separation rely on separation by acidic treatment. In order to investigate the compatibility of the base-mediated deconstruction method, a clear cast block of Airstone^{™} 760E/766H was submerged in acetic acid in a beaker for two months (Fig. 6A). During this time, the block partly fragmented over the course of this time. The solvent was decanted off and the pieces of resin left to dry at room temperature under air for three days (Fig. 6B). The granulate (Fig. 6C) that had gathered at the bottom of the beaker was collected and reacted with 50 wt% of NaOH in toluene under air at 190 °C for 24 h without any further treatment. After an acidic work up and flash column chromatography as previously described, 24.1 mg of BPA were recovered from 100 mg of the fragmented, swollen Airstone^{™} resin. Unfortunately, a yield in percentage cannot be reported, as the swelling of the epoxy resins does not allow approximations regarding the wt% of BPA in the resin. However, the present example nevertheless supports the viability of combining the developed chemical deconstruction with acid-initiated treatments that allow the separation of fibers from resin in fiber-reinforced epoxy composites.

## Claims

1. A method for the deconvolution of epoxy-based polymers (EBPs), the method comprising the steps:
a. providing an EBP,
b. contacting the EBP with a solvent system comprising at least one aprotic apolar organic solvent and an oxygen-containing base to form a suspension, and
c. heating said suspension to a temperature of at least 130°C for at least 2 hours, thereby releasing monomers of the EBPs into the suspension,
wherein the base is added in an amount corresponding to from 20 % to 100 % by weight of the EBP, and wherein the method does not comprise or make use of a transition metal catalyst.

2. The method according to any one of the preceding claims, wherein the EBPs comprise the chemical motif of Formula (III-a) 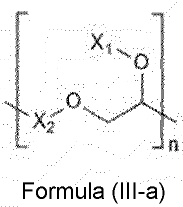 wherein,
X₁ is selected from the group consisting of H, R, or -C(=O)R, wherein R can be C₁₋₆ alkyl, C₁₋₆ heteroalkyl, C₁₋₆ alkoxy, C₁₋₆ allyl, C₁₋₆ heteroallyl, C₄₋₈ cycloalkyl, C₄₋₈ heterocycloalkyl, C₄₋₈ aryl, and C₄₋₈ heteroaryl, each of which may be optionally substituted with one or more of -OH, -NH₂, -L, -CL₃, -OCH₃, wherein L is a halogen;
X₂ is selected from the group consisting of C₄₋₈ cycloalkyl, C₄₋₈ heterocycloalkyl, C₄₋₈ aryl, and C₄₋₈ heteroaryl, each of which may be optionally substituted with one or more of -OH, -NH₂, -Q, -CQ₃, -OCH₃, wherein each of Q is independently hydrogen, halogen, methyl, phenyl or benzyl, preferably wherein X₂ is phenyl; and
n represents the motif as repeating, and may be an integer larger than 1.

3. The method according to any one of the preceding claims, wherein the EBP is an amine-cured epoxy resin, such as wherein the EBPs comprise one or more cross-linkers and/or hardeners selected from the group of poly amide resins, poly(oxypropylene)diamine, 3-aminomethyl-3,5,5-trimethylcyclohexylamine, triethylenetetramines, tetraethylene-pentamines and trimethylolpropane tris[poly(propylene glycol), amine terminated] ether (T-403).

4. The method according to any one of the preceding claims, wherein the EBPs are bisphenol-based EBPs, such as bisphenol A-based EBPs.

5. The method according to any one of the preceding claims, wherein the monomers released into the suspension are bisphenol-based monomers, preferably wherein the monomers released into the suspension are bisphenol A-based monomers, more preferably wherein the monomers released into the suspension are bisphenol A.

6. The method according to any one of the preceding claims, wherein the method allows for direct separation and isolation of bisphenol A-based monomers.

7. The method according to any one of the preceding claims, wherein the EBPs comprise the chemical motif of formula (III-ca): 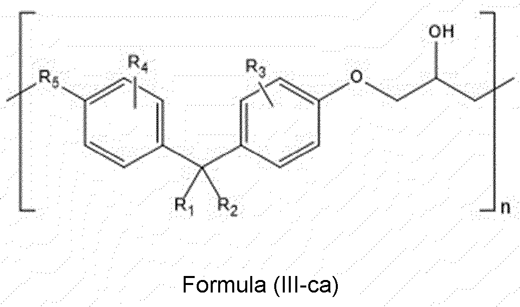 wherein the motif of Formula (III-ca) may comprise from 1 to 4 of each of R₃ and R₄, and wherein R₁, R₂, R₃, and R₄ are each individually selected from the group consisting of H, C₁₋₆ alkyl, C₁₋₆ alkenyl, CF₃, F, Cl, Br, OH, NO₂, NH₂, phenyl, or wherein R₁ and R₂ may come together to form a C₆-cycloalkyl, and wherein R₅ is selected as O or NZ, wherein NZ represents an amine based crosslinker and/or hardener selected from the group consisting of poly amide resins, poly(oxypropylene)diamine, 3-aminomethyl-3,5,5-trimethylcyclohexylamine, triethylenetetramines, tetraethylene-pentamines, and trimethylolpropane tris[poly(propylene glycol), amine terminated] ether (T-403).

8. The method according to any one of the preceding claims, wherein the suspension is heated to a temperature between 130 °C to 250 °C, such as a temperature between 170 °C and 250 °C, or a temperature between 170 °C and 210 °C.

9. The method according to any one of the preceding claims, wherein the organic solvent is selected from the group consisting of toluene, benzene, xylene, mesitylene, cumene, cymene, and xylol, or a structural isomer of any one of these, preferably wherein the solvent is selected from toluene, xylene and cymene, or a structural isomer of any one of these, more preferably wherein the organic solvent is selected from the group consisting of toluene, p-cymene, o-xylene .

10. The method according to any one of the preceding claims, wherein the oxygen-containing base is a hydroxide selected from the group consisting of NaOH, LiOH, KOH, CsOH, RbOH, Ca(OH)₂, Sr(OH)₂, and Ba(OH)₂; or an alkoxide selected from the group consisting of n-butoxide, tert-butoxide, methoxide, ethoxides, and isopropoxide, including sodium or potassium salts of these, preferably wherein the base is selected from the group consisting of NaOH, KOH, NaOBu, and NaOtBu.

11. The method according to any one of the preceding claims, wherein the base is a nucleophile base, and/or the base is not a nitrogen-containing base.

12. The method according to any one of the preceding claims, wherein the method is:
a) conducted in a closed vessel under autogenous pressure, such as performed in a closed vessel under an autogenous pressure of 0.10 MPa to 1.00 MPa (14.5 to 145 psi); and/or
b) performed in an inert atmosphere, such as an atmosphere consisting essentially of nitrogen (N₂) or Argon (Ar); or in a non-inert atmosphere, such as using ambient air.

13. The method according to any one of the preceding claims, wherein the method is a one pot reaction.

14. The method according to any one of the preceding claims, wherein the epoxy-based polymer may be mixed with fibres, such as in fibre-reinforced composites, in particular those where the fibres are glass fibre or carbon fibre.

## Patentansprüche

1. Verfahren zur Dekonvolution von epoxidbasierten Polymeren (EBPs), wobei das Verfahren die Schritte umfasst:
a. Bereitstellen eines EBP,
b. Inkontaktbringen des EBP mit einem Lösungsmittelsystem, das mindestens ein aprotisches apolares organisches Lösungsmittel und eine sauerstoffhaltige Base umfasst, um eine Suspension zu bilden, und
c. Erhitzen der Suspension auf eine Temperatur von mindestens 130 °C für mindestens 2 Stunden, wodurch Monomere der EBPs in die Suspension freigesetzt werden,
wobei die Base in einer Menge zugegeben wird, die 20 Gew.-% bis 100 Gew.-% des EBP entspricht, und wobei das Verfahren keinen Übergangsmetallkatalysator umfasst oder verwendet.

2. Verfahren nach einem der vorhergehenden Ansprüche, wobei die EBPs das chemische Motiv der Formel (III-a) umfassen 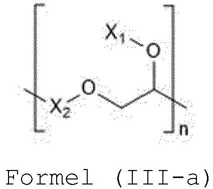 wobei,
X₁ ausgewählt ist aus der Gruppe bestehend aus H, R oder - C(=O)R, wobei R C₁₋₆-Alkyl, C₁₋₆-Heteroalkyl, C₁₋₆-Alkoxy, C₁₋₆-Allyl, C₁₋₆-Heteroallyl, C₄₋₈-Cycloalkyl, C₄₋₈-Heterocycloalkyl, C₄₋₈-Aryl und C₄₋₈-Heteroaryl sein kann, von denen jedes optional substituiert sein kann mit einem oder mehreren von - OH, -NH₂, -L, -CL₃, -OCH₃, wobei L ein Halogen ist;
X₂ ausgewählt ist aus der Gruppe bestehend aus C₄₋₈-Cycloalkyl, C₄₋₈-Heterocycloalkyl, C₄₋₈-Aryl und C₄₋₈-Heteroaryl, von denen jedes optional substituiert sein kann mit einem oder mehreren von -OH, -NH₂, -Q, -CQ₃, -OCH₃, wobei jedes Q unabhängig Wasserstoff, Halogen, Methyl, Phenyl oder Benzyl ist, vorzugsweise wobei X₂ Phenyl ist; und
n das Motiv als sich wiederholend darstellt und eine ganze Zahl größer als 1 sein kann.

3. Verfahren nach einem der vorhergehenden Ansprüche, wobei das EBP ein amingehärtetes Epoxidharz ist, beispielsweise wobei die EBPs einen oder mehrere Vernetzer und/oder Härter umfassen, ausgewählt aus der Gruppe von Polyamidharzen, Poly(oxypropylen)diamin, 3-Aminomethyl-3,5,5-trimethylcyclohexylamin, Triethylentetraminen, Tetraethylenpentaminen und Trimethylolpropan-tris[poly(propylenglykol), aminterminiert]-ether (T-403).

4. Verfahren nach einem der vorhergehenden Ansprüche, wobei die EBPs bisphenolbasierte EBPs sind, wie Bisphenol-A-basierte EBPs.

5. Verfahren nach einem der vorhergehenden Ansprüche, wobei die in die Suspension freigesetzten Monomere bisphenolbasierte Monomere sind, vorzugsweise wobei die in die Suspension freigesetzten Monomere Bisphenol-A-basierte Monomere sind, besonders bevorzugt wobei die in die Suspension freigesetzten Monomere Bisphenol A sind.

6. Verfahren nach einem der vorhergehenden Ansprüche, wobei das Verfahren eine direkte Trennung und Isolierung von Bisphenol-A-basierten Monomeren ermöglicht.

7. Verfahren nach einem der vorhergehenden Ansprüche, wobei die EBPs das chemische Motiv der Formel (III-ca) umfassen: 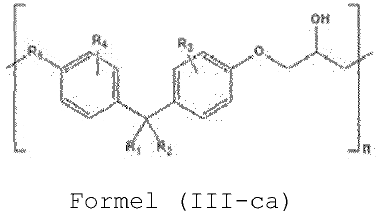 wobei das Motiv der Formel (III-ca) jeweils 1 bis 4 von R₃ und R₄ umfassen kann, und wobei R₁, R₂, R₃ und R₄ jeweils einzeln ausgewählt sind aus der Gruppe bestehend aus H, C₁₋₆-Alkyl, C₁₋₆-Alkenyl, CF₃, F, Cl, Br, OH, NO₂, NH₂, Phenyl, oder wobei R₁ und R₂ zusammen ein C₆-Cycloalkyl bilden können, und wobei R₅ als O oder NZ ausgewählt ist, wobei NZ einen aminbasierten Vernetzer und/oder Härter darstellt, ausgewählt aus der Gruppe bestehend aus Polyamidharzen, Poly(oxypropylen)diamin, 3-Aminomethyl-3,5,5-trimethylcyclohexylamin, Triethylentetraminen, Tetraethylenpentaminen und Trimethylolpropan-tris[poly(propylenglykol), aminterminiert]-ether (T-403).

8. Verfahren nach einem der vorhergehenden Ansprüche, wobei die Suspension auf eine Temperatur zwischen 130 °C und 250 °C erhitzt wird, wie eine Temperatur zwischen 170 °C und 250 °C, oder eine Temperatur zwischen 170 °C und 210 °C.

9. Verfahren nach einem der vorhergehenden Ansprüche, wobei das organische Lösungsmittel ausgewählt ist aus der Gruppe bestehend aus Toluol, Benzol, Xylol, Mesitylen, Cumol, Cymol und Xylol, oder einem Strukturisomer eines dieser, vorzugsweise wobei das Lösungsmittel ausgewählt ist aus Toluol, Xylol und Cymol, oder einem Strukturisomer eines dieser, besonders bevorzugt wobei das organische Lösungsmittel ausgewählt ist aus der Gruppe bestehend aus Toluol, *p*-Cymol, *o*-Xylol.

10. Verfahren nach einem der vorhergehenden Ansprüche, wobei die sauerstoffhaltige Base ein Hydroxid ist, ausgewählt aus der Gruppe bestehend aus NaOH, LiOH, KOH, CsOH, RbOH, Ca(OH)₂, Sr(OH)₂ und Ba(OH)₂; oder ein Alkoxid, ausgewählt aus der Gruppe bestehend aus n-Butoxid, *tert*-Butoxid, Methoxid, Ethoxiden und Isopropoxid, einschließlich Natrium- oder Kaliumsalzen dieser, vorzugsweise wobei die Base ausgewählt ist aus der Gruppe bestehend aus NaOH, KOH, NaOBu und NaO*t*Bu.

11. Verfahren nach einem der vorhergehenden Ansprüche, wobei die Base eine nucleophile Base ist und/oder die Base keine stickstoffhaltige Base ist.

12. Verfahren nach einem der vorhergehenden Ansprüche, wobei das Verfahren:
a) in einem geschlossenen Gefäß unter autogenem Druck durchgeführt wird, wie in einem geschlossenen Gefäß unter einem autogenen Druck von 0,10 MPa bis 1,00 MPa (14,5 bis 145 psi) durchgeführt wird; und/oder
b) in einer Inertatmosphäre durchgeführt wird, wie einer Atmosphäre, die im Wesentlichen aus Stickstoff (N₂) oder Argon (Ar) besteht; oder in einer nicht inerten Atmosphäre, wie unter Verwendung von Umgebungsluft.

13. Verfahren nach einem der vorhergehenden Ansprüche, wobei das Verfahren eine Eintopfreaktion ist.

14. Verfahren nach einem der vorhergehenden Ansprüche, wobei das epoxidbasierte Polymer mit Fasern gemischt sein kann, wie in faserverstärkten Verbundwerkstoffen, insbesondere solchen, bei denen die Fasern Glasfaser oder Kohlenstofffaser sind.

## Revendications

1. Procédé pour le désassemblage de polymères à base d'époxydes (EBP), le procédé comprenant les étapes suivantes :
a. fourniture d'un EBP,
b. mise en contact de l'EBP avec un système de solvants comprenant au moins un solvant organique aprotique apolaire et une base contenant de l'oxygène pour former une suspension, et
c. chauffage de ladite suspension à une température d'au moins 130 °C pendant au moins 2 heures, libérant ainsi les monomères des EBP dans la suspension,
dans lequel la base est ajoutée en une proportion correspondant à de 20 % à 100 % en poids de l'EBP, et dans lequel le procédé ne comprend ni n'utilise de catalyseur à métal de transition.

2. Procédé selon l'une quelconque des revendications précédentes, dans lequel les EBP comprennent le motif chimique de formule (III-a) 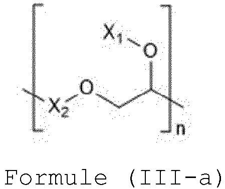 dans lequel,
X₁ est choisi dans le groupe constitué par H, R ou -C(=O)R, dans lequel R peut être un alkyle en C₁₋₆, un hétéroalkyle en C₁₋₆, un alcoxy en C₁₋₆, un allyle en C₁₋₆, un hétéroallyle en C₁₋₆, un cycloalkyle en C₄₋₈, un hétérocycloalkyle en C₄₋₈, un aryle en C₄₋₈ et un hétéroaryle en C₄₋₈, chacun de ceux-ci pouvant être éventuellement substitué par un ou plusieurs groupes parmi -OH, -NH₂, -L, -CL₃, -OCH₃, dans lequel L représente un halogène ;
X₂ est choisi dans le groupe constitué par un cycloalkyle en C₄₋₈, un hétérocycloalkyle en C₄₋₈, un aryle en C₄₋₈ et un hétéroaryle en C₄₋₈, chacun de ceux-ci pouvant être éventuellement substitué par un ou plusieurs groupes parmi -OH, -NH₂, -Q, -CQ₃, -OCH₃, dans lequel chaque Q représente indépendamment un hydrogène, un halogène, un méthyle, un phényle ou un benzyle, dans lequel de préférence X₂ représente un phényle ; et
n représente le motif comme motif répété, et peut être un entier supérieur à 1.

3. Procédé selon l'une quelconque des revendications précédentes, dans lequel l'EBP est une résine époxyde durcie par des amines, tel que dans lequel les EBP comprennent un ou plusieurs agents de réticulation et/ou durcisseurs choisis dans le groupe constitué par des résines polyamides, une poly(oxypropylène)diamine, une 3-aminométhyl-3,5,5-triméthylcyclohexylamine, des triéthylènetétramines, des tétraéthylène-pentamines et un éther de tris[poly(propylène glycol), à terminaison amine] de triméthylolpropane (T-403).

4. Procédé selon l'une quelconque des revendications précédentes, dans lequel les EBP sont des EBP à base de bisphénol, tels que des EBP à base de bisphénol A.

5. Procédé selon l'une quelconque des revendications précédentes, dans lequel les monomères libérés dans la suspension sont des monomères à base de bisphénol, de préférence dans lequel les monomères libérés dans la suspension sont des monomères à base de bisphénol A, plus préférablement dans lequel les monomères libérés dans la suspension sont du bisphénol A.

6. Procédé selon l'une quelconque des revendications précédentes, dans lequel le procédé permet la séparation directe et l'isolement de monomères à base de bisphénol A.

7. Procédé selon l'une quelconque des revendications précédentes, dans lequel les EBP comprennent le motif chimique de formule (III-ca) : 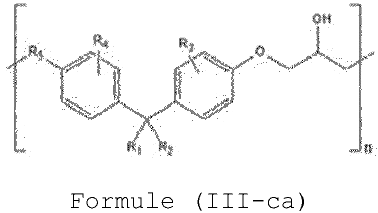 dans lequel le motif de formule (III-ca) peut comprendre de 1 à 4 de chacun de R₃ et R₄, et dans laquelle R₁, R₂, R₃ et R₄ sont chacun choisis individuellement dans le groupe constitué par H, un alkyle en C₁₋₆, un alcényle en C₁₋₆, CF₃, F, Cl, Br, OH, NO₂, NH₂, un phényle, ou dans lequel R₁ et R₂ peuvent être reliés pour former un cycloalkyle en C₆, et dans lequel R₅ est choisi parmi O ou NZ, dans lequel NZ représente un agent de réticulation et/ou un durcisseur à base d'amine choisi dans le groupe constitué par des résines polyamides, une poly(oxypropylène)diamine, une 3-aminométhyl-3,5,5-triméthylcyclohexylamine, des triéthylènetétramines, des tétraéthylène-pentamines et un éther de tris[poly(propylène glycol), à terminaison amine] de triméthylolpropane (T-403).

8. Procédé selon l'une quelconque des revendications précédentes, dans lequel la suspension est chauffée à une température comprise entre 130 °C et 250 °C, telle qu'une température comprise entre 170 °C et 250 °C, ou une température comprise entre 170 °C et 210 °C.

9. Procédé selon l'une quelconque des revendications précédentes, dans lequel le solvant organique est choisi dans le groupe constitué par le toluène, le benzène, le xylène, le mésitylène, le cumène, le cymène et le xylol, ou un isomère structural de l'un quelconque de ceux-ci, de préférence dans lequel le solvant est sélectionné parmi le toluène, le xylène et le cymène, ou un isomère structural de l'un quelconque de ceux-ci, plus préférablement dans lequel le solvant organique est sélectionné dans le groupe constitué par le toluène, le *p-*cymène et l'*o*-xylène.

10. Procédé selon l'une quelconque des revendications précédentes, dans lequel la base contenant de l'oxygène est un hydroxyde choisi dans le groupe constitué par NaOH, LiOH, KOH, CsOH, RbOH, Ca(OH)₂, Sr(OH)₂ et Ba(OH)₂ ; ou un alcoxyde choisi dans le groupe constitué par un n-butoxyde, un *tert-*butoxyde, un méthoxyde, des éthoxydes et un isopropoxyde, y compris des sels de sodium ou de potassium de ceux-ci, de préférence dans lequel la base est choisie dans le groupe constitué par NaOH, KOH, NaOBu et NaO*t*Bu.

11. Procédé selon l'une quelconque des revendications précédentes, dans lequel la base est une base nucléophile et/ou la base n'est pas une base contenant de l'azote.

12. Procédé selon l'une quelconque des revendications précédentes, dans lequel le procédé est :
a) effectué dans un récipient fermé sous pression autogène, tel qu'effectué dans un récipient fermé sous une pression autogène de 0,10 MPa à 1,00 MPa (de 14,5 à 145 psi) ; et/ou
b) effectué sous une atmosphère inerte, telle qu'une atmosphère composée essentiellement d'azote (N₂) ou d'argon (Ar) ; ou sous une atmosphère non inerte, telle qu'avec de l'air ambiant.

13. Procédé selon l'une quelconque des revendications précédentes, dans lequel le procédé est une réaction monotope.

14. Procédé selon l'une quelconque des revendications précédentes, dans lequel le polymère à base d'époxyde peut être mélangé avec des fibres, tel que dans des composites renforcés par des fibres, en particulier ceux dans lesquels les fibres sont des fibres de verre ou des fibres de carbone.
